# EUROPEAN PATENT APPLICATION

(11) **EP 4 644 354 A1**
(43) Date of publication of application: **05.11.2025**
(21) Application number: 23911747.6
(22) Date of filing: 14.12.2023
(51) Int. Cl.: C07B 61/00, C07B 31/00, C07C 5/00, C07C 9/15, C07C 13/567, C07C 13/615, C07C 15/14, C07C 15/24

(54) **POLYMER MECHANORADICAL INITIATOR AND REACTION METHOD USING POLYMER MECHANORADICAL INITIATOR**

(30) Priority: 28.12.2022 JP 2022211011
(71) Applicant: National University Corporation Hokkaido University, Hokkaido 060-0808 (JP)
(72) Inventor: ITO, Hajime, Sapporo-shi, Hokkaido 060-0808 (JP); KUBOTA, Koji, Sapporo-shi, Hokkaido 060-0808 (JP)
(74) Representative: Hasegawa, Kan
(86) International application number: PCT/JP2023/044838
(87) International publication number: WO 2024/142966

(57) **Abstract**

An object is to provide a polymer mechanoradical initiator obtained by mechanochemical treatment of an organic polymer material, the polymer mechanoradical initiator being used as a radical initiator for a reaction in which a polymer mechanoradical initiator component is not incorporated into a reaction product, to provide a reaction method for radical reduction of an organohalogen compound using a polymer mechanoradical initiator obtained by mechanochemical treatment of an organic polymer material, to provide a reaction method for radical cyclization of an organohalogen compound using a polymer mechanoradical initiator obtained by mechanochemical treatment of an organic polymer material, or to provide a reaction method for C-H fluorination of a compound having a C-H bond based on secondary carbon or tertiary carbon using a polymer mechanoradical initiator obtained by mechanochemical treatment of an organic polymer material. As solutions, the following are provided: (i) a polymer mechanoradical initiator obtained by mechanochemical treatment of an organic polymer material, in which the polymer mechanoradical initiator is used in a reaction in which the polymer mechanoradical initiator is not incorporated into a reaction product; (ii) a reaction method for radical reduction and/or radical cyclization of a substrate using a polymer mechanoradical initiator, the method including a step of performing mechanochemical treatment of at least an organic polymer material and a substrate in a reaction vessel, in which the substrate contains an organohalogen compound; and (iii) a reaction method for C-H fluorination of a substrate using a polymer mechanoradical initiator, the method including a step of performing mechanochemical treatment of at least an organic polymer material, a substrate, and a fluorinating agent in a reaction vessel, in which the substrate contains a compound having a C-H bond based on secondary carbon or tertiary carbon.

## Description

### Technical Field

The present invention relates to a polymer mechanoradical initiator. The present invention relates to a radical reduction reaction method, a radical cyclization reaction method, and a C-H fluorination reaction method using a polymer mechanoradical initiator.

### Background Art

It is known that a chain-type radical reaction using a radical initiator is used in the synthesis of functional materials, such as medicines, liquid crystal compounds, organic electroluminescence compounds, organic thin-film solar cells, polymer compounds, oligomers, coloring materials, energy-ray-absorbing materials, information-recording materials, wavelength conversion materials, indicator materials, sensor materials, organic light-emitting diodes (OLEDs), and organic semiconductor materials. In order to generate a chain-type radical reaction, it is necessary to obtain a radical species. Typically, radical initiators, such as azobisisobutyronitrile (AIBN), are used. However, most radical initiators are unstable to heat and light, and many of them have explosiveness and the like and are not satisfactory in terms of stability, handleability, practicality, and the like. Thus, there is a demand for a radical initiator having excellent stability, handleability, and the like and having practicality.

Patent Literature 1 discloses that a cellulose ester is subjected to mechanochemical treatment to generate a cellulose mechanoradical, and the cellulose mechanoradical is allowed to react with a (meth)acrylic monomer to obtain a block copolymer made from the cellulose ester and the (meth)acrylic monomer.

Patent Literature 2 and Non Patent Literature 1 disclose that after generating a polymer mechanoradical, a light-emitting compound is bonded, and the resulting product is used as a light-emitting polymer or a radical detection probe.

### Citation List

### Patent Literature

PTL 1: Japanese Unexamined Patent Application Publication No. 2012-88358
PTL 2: Japanese Unexamined Patent Application Publication No. 2021-173603

### Non Patent Literature

NPL 1: K. Kubota, et al, Angew. Chem. Int. Ed., 2021, Vol. 60, No. 29, 16003-16008

### Summary of Invention

### Technical Problem

Polymer mechanoradical initiators are known to be radical initiators having excellent stability, handleability, and the like and practicality. However, polymer mechanoradical initiators have been used so far in reactions, such as block polymerization and polymer modification, in which a polymer is used as a substrate and the polymer is incorporated into the reaction product, and have not been commonly used in other radical reactions, such as chain-type radical reactions.

To solve the above problems, it is an object of the present invention to provide a polymer mechanoradical initiator obtained by mechanochemical treatment of an organic polymer material, the polymer mechanoradical initiator being used as a radical initiator for a reaction in which a polymer mechanoradical initiator component is not incorporated into a reaction product.

To solve the above problems, it is an object of the present invention to provide a reaction method for radical reduction of an organohalogen compound using a polymer mechanoradical initiator obtained by mechanochemical treatment of an organic polymer material as a radical initiator.

To solve the above problems, it is an object of the present invention to provide a reaction method for radical cyclization of an organohalogen compound using a polymer mechanoradical initiator obtained by mechanochemical treatment of an organic polymer material as a radical initiator.

To solve the above problems, it is an object of the present invention to provide a reaction method for C-H fluorination of a compound having a C-H bond based on secondary carbon or tertiary carbon using a polymer mechanoradical initiator obtained by mechanochemical treatment of an organic polymer material.

### Solution to Problem

The inventors have conducted intensive studies, have found that the above problems can be solved by the following (a) to (c), and have completed the present invention:
(a) a polymer mechanoradical initiator for use in a particular reaction;
(b) a reaction method for radical reduction and/or radical cyclization of a substrate using a polymer mechanoradical initiator, the reaction method including a specific step, in which the substrate contains an organohalogen compound; and
(c) a reaction method for C-H fluorination of a substrate using a polymer mechanoradical initiator, the reaction method including a specific step, in which the substrate is a compound having a C-H bond based on secondary carbon or tertiary carbon.

That is, the present invention is as described below.
[Item 1] A polymer mechanoradical initiator obtained by mechanochemical treatment of an organic polymer material, in which the polymer mechanoradical initiator is used in a reaction in which the polymer mechanoradical initiator is not incorporated into a reaction product.
[Item 2] The radical initiator described in item 1, in which the organic polymer material contains a polyolefin-based material, a polyaromatic vinyl-based material, a polyvinyl ether-based material, a polyvinyl ester-based material, a (meth)acrylic resin-based material, a cellulose-based material, a polyether-based material, a polyester-based material, a polycarbonate-based material, a polyketone-based material, a polyurethane-based material, a polyamide-based material, a polyimide-based material, a polysiloxane-based material, a polyacetal-based material, a modified polyphenylene ether-based material, an ABS resin-based material, and a halogenated olefin-based material.
[Item 3] A reaction method for radical reduction and/or radical cyclization of a substrate using a polymer mechanoradical initiator, the method including:
   a step of performing mechanochemical treatment of at least an organic polymer material and a substrate in a reaction vessel,
   in which the substrate contains an organohalogen compound.
[Item 4] A reaction method for C-H fluorination of a substrate using a polymer mechanoradical initiator, the method including:
   a step of performing mechanochemical treatment of at least an organic polymer material, a substrate, and a fluorinating agent in a reaction vessel,
   in which the substrate contains a compound having a C-H bond based on secondary carbon or tertiary carbon.
[Item 5] The reaction method described in item 3 or 4, in which the organic polymer material contains a polyolefin-based material, a polyaromatic vinyl-based material, a polyvinyl ether-based material, a polyvinyl ester-based material, a (meth)acrylic resin-based material, a cellulose-based material, a polyether-based material, a polyester-based material, a polycarbonate-based material, a polyketone-based material, a polyurethane-based material, a polyamide-based material, a polyimide-based material, a polysiloxane-based material, a polyacetal-based material, a modified polyphenylene ether-based material, an ABS resin-based material, and a halogenated olefin-based material.
[Item 6] The reaction method described in any one of items 3 to 5, in which the organic polymer material and/or the substrate includes waste.
[Item 7] The reaction method described in item 3, 5, or 6, in which the reaction method is a radical reduction reaction method, and a reducing agent containing a silane-based reducing agent and/or a tin-based reducing agent is used. Advantageous Effects of Invention

According to the present invention, a polymer mechanoradical initiator obtained by mechanochemical treatment of an organic polymer material is provided as a radical initiator for a reaction in which a polymer mechanoradical initiator component is not incorporated into a reaction product.

According to the present invention, a reaction method for radical reduction of an organohalogen compound is provided, in which a polymer mechanoradical initiator obtained by the mechanochemical treatment of an organic polymer material is used as a radical initiator.

According to the present invention, a reaction method for radical cyclization of an organohalogen compound is provided, in which a polymer mechanoradical initiator obtained by the mechanochemical treatment of an organic polymer material is used as a radical initiator.

According to the present invention, a reaction method for C-H fluorination of a compound having a C-H bond based on secondary carbon or tertiary carbon is provided, in which a polymer mechanoradical initiator obtained by mechanochemical treatment of an organic polymer material is used.

### Description of Embodiments

A first aspect of the present invention is a polymer mechanoradical initiator obtained by mechanochemical treatment of an organic polymer material, in which the polymer mechanoradical initiator is used for a reaction in which a polymer mechanoradical initiator component is not incorporated into a reaction product.

A second aspect of the present invention is a reaction method for radical reduction and/or radical cyclization of a substrate using a polymer mechanoradical initiator, the method including a step of performing mechanochemical treatment of at least an organic polymer material and a substrate in a reaction vessel, in which the substrate contains an organohalogen compound.

When the reaction method is a radical reduction reaction method, a reducing agent including a silane-based reducing agent and/or a tin-based reducing agent can be used.

A third aspect of the present invention is a reaction method for C-H fluorination of a substrate using a polymer mechanoradical initiator, the method including a step of performing mechanochemical treatment of at least an organic polymer material, a substrate, and a fluorinating agent in a reaction vessel, in which the substrate contains a compound having a C-H bond based on secondary carbon or tertiary carbon.

Details will be described below.

### [Organic Polymer Material]

In each of the first to third aspects of the present invention, the organic polymer material to be subjected to mechanochemical treatment is not particularly limited. For example, one or more types selected from the group consisting of, for example, polymer materials obtained by addition polymerization of compounds having carbon-carbon unsaturated bonds, such as vinyl-based monomers, and polymer materials obtained by polycondensation reactions can be used. Examples of the material include one or more types selected from the group consisting of polyolefin-based materials, polyvinyl ether-based materials, polyaromatic vinyl-based materials, polyvinyl ester-based materials, (meth)acrylic resin-based materials, fluorinated polyolefin-based materials, polyvinyl chloride-based materials, polyvinyl alcohol-based materials, polyvinyl amine-based materials, graft polymer-based materials based on addition polymerization of compounds having carbon-carbon unsaturated bonds, block polymer-based materials based on compounds having carbon-carbon unsaturated bonds, cellulose-based materials, polyether-based materials, polyester-based materials, polycarbonate-based materials, polyketone-based materials, polyurethane-based materials, polyamide-based materials, polyimide-based materials, polysiloxane-based materials, polyacetal-based materials, modified polyphenylene ether-based materials, ABS resin-based materials, and the like.

The molecular weight (weight-average molecular weight) of the organic polymer material to be subjected to mechanochemical treatment is not particularly limited. For example, the molecular weight is 3,000 or more, preferably 5,000 or more, and for example, 10,000,000 or less, preferably 7,000,000 or less.

In each of the first to third aspects of the present invention, the organic polymer material preferably contains one or more types of polymer materials obtained by addition polymerization of compounds having carbon-carbon unsaturated bonds, such as vinyl-based monomers. Among these, one or more types selected from the group consisting of polyolefin-based materials, polyaromatic vinyl-based materials, polyvinyl ester-based materials, (meth)acrylic resin-based materials, polyester-based materials, polycarbonate-based materials, polyamide-based materials, polyurethane-based materials, polyacetal-based materials, modified polyphenylene ether-based materials, ABS resin-based materials, and halogenated olefin-based materials are more preferably contained.

In addition, the organic polymer material may also include waste plastics, which are waste after use. Waste plastics refer to all types of waste plastic products, and may be either industrial waste or general waste.

Industrial waste is plastic waste discharged from industries related to resin materials (plastic products), such as resin material manufacturers and plastic molding material manufacturers. Examples thereof include one or more types selected from the group consisting of olefin-based materials, such as polyethylene-based resins, polypropylene-based resins, and copolymers made mainly from olefin-based monomers; polyaromatic vinyl-based materials, such as polystyrene-based polymers; polyvinyl ester-based materials, such as polyvinyl acetate-based polymers; (meth)acrylic resin-based materials, such as (meth)acrylate-based polymers; and others, such as AS resins, ABS resins, polyvinyl-based materials, e.g., polyvinyl chloride-based resins, engineering plastics, e.g., polyamide, polyester, polyacetal, polycarbonate, and polyphenylene ether, and rubber materials, e.g., ethylene-propylene-based copolymers and styrene-butadiene-styrene copolymers.

General waste is plastic waste discharged from general households, and examples thereof include plastic packaging materials and plastic everyday items that are made from the same materials as industrial waste.

### [Mechanochemical Treatment]

In each of the first to third aspects of the present invention, the mechanochemical treatment is treatment for cutting the polymer chain of the organic polymer material by directly applying mechanical energy generated mechanically by means of, for example, grinding, shearing, impact, or compression to the organic polymer material, thereby generating a polymer mechanoradical. The mechanochemical treatment can be performed under solvent-free or substantially solvent-free conditions; thus, it contributes to a reduction in waste and has a low environmental impact, and it also does not require the setting of complicated reaction conditions or preparation. Furthermore, it is possible to generate a polymer radical easily and in a very short time, and it is possible to achieve both reduced energy consumption and high productivity.

The following reaction device and the like used in the mechanochemical treatment and the following various conditions and the like in the mechanochemical treatment can be used.

### <Reaction Device>

The reaction device used in the mechanochemical treatment is not particularly limited as long as it is a device that can apply mechanical energy to the organic polymer material to thereby obtain the polymer mechanoradical initiator and that can perform a reaction using the polymer mechanoradical initiator.

Examples of such a device include one or more types selected from the group consisting of, for example:
mills, such as ball mills, rod mills, jet mills, and SAG mills;
grinders, such as rotary millstones and automated mortars;
(horizontal-axis rotating) rotary vessel-type mixing devices, such as the horizontal cylinder type, the V type, the double-cone type, the rectangular-cubic type, the S type, and the continuous V type;
rotary vessel-type mixing devices (with baffle plates and blades), such as the horizontal cylindrical type, the V type, the double-cone type, and the ball mill type;
(rotationally oscillating) rotary vessel-type mixing devices, such as the rocking type and the cross-rotary type;
(horizontal-axis rotating) fixed vessel-type mixing devices, such as the ribbon type, the paddle type, the single-axis rotor type, and the bug mill type;
(vertical-axis rotating) fixed vessel-type mixing devices, such as the ribbon type, the screw type, the planetary type, the turbine type, the high-speed flow type, the rotating disk type, and the muller type;
(vibratory) fixed vessel-type mixing devices, such as the vibratory mill type and sieves;
(fluidization) fluid motion-type mixing devices, such as the heterogeneous fluidized bed type, the swirling fluidized bed type, the riser tube type, and the jet pump type;
(gravitational) fluid motion-type mixing devices, such as the gravity type and static mixers; and
kneaders, such as twin-screw kneaders, single-screw kneaders, mixers, and roll mills.

As the device used in the mechanochemical treatment, preferably, one or more types selected from the group consisting of a mill, a grinder, a mixing device, a kneader, and the like is used, and particularly preferably, a mixing device is used. For the mixing device, for example, reference can be made to the powder mixing devices described in, for example, Table 5 and Fig. 9 in Sakashita, "Mixture Process Technology for the Powders" Journal of the Japan Society of Colour Material, 77(2), 75-85(2004). Specifically, a ball mill, a twin-screw kneader, a planetary ball mill, a SPEX mixer mill, a twin-shaft ball mill, or the like can be used.

A device used in the mechanochemical treatment may further include at least one or more types selected from the group consisting of measuring means, depressurizing or pressurizing means, atmosphere-adjusting means (gas introduction or exhaustion means), means for introducing various components, means for taking out various components or reaction products, purification means, analysis means, and the like.

### <Reaction Vessel>

The reaction vessel used in the mechanochemical treatment is not particularly limited. An appropriate reaction vessel is selected and used in consideration of the physical properties, reactivity, and amounts of the organic polymer material, the substrate for a reaction using the polymer mechanoradical initiator, and other components present in the reaction system, reaction conditions for the mechanochemical treatment, and reaction conditions for the reaction using the polymer mechanoradical initiator. For example, when a device for mechanically performing mixing treatment (for example, a ball mill) is used, a ball mill jar or the like can be used as the reaction vessel.

The reaction vessel used in the mechanochemical treatment may be equipped with stirring means for stirring components, such as the organic polymer material and the substrate that contributes to the reaction of the polymer mechanoradical initiator. The stirring means with which the reaction vessel may be equipped is not particularly limited as long as it is any of the various stirring means with which the reaction device can be equipped. The above-described (Reaction Device) can be used as means for mechanically performing mixing treatment. As a device for mechanically performing mixing treatment, for example, a ball mill is preferably used.

### <Conditions for Mechanochemical Treatment>

Conditions for the mechanochemical treatment are not particularly limited. Appropriate conditions are used in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator.

When the organic polymer material is subjected to mechanochemical treatment under appropriate conditions, the main chain of the organic polymer is subjected to homolytic cleavage by the mechanical action of the mechanochemical treatment, making it possible to easily generate polymer mechanoradicals.

### (Mechanical Energy to Be Applied)

In the mechanochemical treatment, the mechanical energy to be applied is not particularly limited as long as it is mechanical energy with which at least a polymer radical initiator can be formed.

For example, when the device used in the mechanochemical treatment is a mixing device, the mixing speed is not particularly limited. The mixing speed can be appropriately specified in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator. For example, when a ball mill is used, shaking and stirring can be performed at a vibration frequency of 5 Hz or more, preferably 10 Hz or more, and more preferably 20 Hz or more.

### (Temperature)

In the mechanochemical treatment, the temperature in the reaction vessel (the temperature of the reaction system) is not particularly limited as long as it is a temperature at which at least a polymer radical initiator can be formed. The temperature can be appropriately specified in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator. The temperature is, for example, 20°C or higher, preferably 50°C or higher, and more preferably 60°C or higher, and is, for example, 500°C or lower, preferably 300°C or lower, and more preferably 250°C or lower.

A method for controlling the temperature is not particularly limited. Various temperature control methods used in conducting chemical reactions can be used. Examples thereof include a method for controlling the temperature in the reaction vessel (reaction system) by heating or cooling the reaction vessel itself using heating or cooling means; a method for controlling the temperature in the reaction vessel by covering the reaction vessel with a heat medium at a predetermined temperature; and a method for controlling the temperature in the reaction vessel by providing a heating element or a cooling element. From the viewpoints of safety and ease of temperature control operation, a specific example thereof is a method of controlling the temperature in the reaction vessel by applying hot air generated by a heat gun to the reaction vessel.

### (Pressure)

In the mechanochemical treatment, the pressure in the reaction vessel (pressure of the reaction system) is not particularly limited as long as it is a pressure at which at least a polymer radical initiator can be formed. The pressure can be appropriately specified in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator. For example, any of a substantially vacuum state, a reduced pressure state, a pressurized state, and an atmospheric pressure state may be used. When the mechanochemical treatment can be performed in an atmospheric pressure state without pressurizing or depressurizing, the atmospheric pressure state is preferred from the viewpoints of operability, reaction vessels, cost, safety, and the like.

A method for controlling the pressure is not particularly limited. When the reaction vessel (reaction system) is pressurized or depressurized, various pressure control methods used in performing chemical reactions can be used. For example, the pressure in the reaction vessel (pressure of the reaction system) can be increased or decreased using pressurizing or depressurizing means.

### (Atmosphere)

In the mechanochemical treatment, the atmosphere in the reaction vessel (the atmosphere of the reaction system) is not particularly limited as long as it is an atmosphere in which at least a polymer radical initiator can be formed. The atmosphere can be appropriately specified in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator. For example, the treatment can be performed in an air atmosphere without any particular adjustment of the atmosphere. If necessary, the treatment can be performed in an inert gas atmosphere, such as nitrogen, helium, neon, or argon.

### (Treatment Time)

In the mechanochemical treatment, the treatment time is not particularly limited as long as it is a treatment time during which at least a polymer radical initiator can be formed. The treatment time can be appropriately specified in consideration of the physical properties, reactivity, and amounts of the organic polymer material, the substrate for the reaction using the polymer mechanoradical initiator, and other components present in the reaction system, the reaction conditions for the mechanochemical treatment, and the reaction conditions for the reaction using the polymer mechanoradical initiator. For example, the treatment time can be 1 minute or more, preferably 3 minutes or more, and more preferably 5 minutes or more. The upper limit of the reaction time is not particularly limited, but can be, for example, 10 hours or less, preferably 5 hours or less, and more preferably 3 hours or less.

### (Addition Order and the Like of Substance to Be Treated and Substrate During Treatment, and Treatment and the Like After Reaction)

In the mechanochemical treatment, the order in which the organic polymer material, the substrate for the reaction using the polymer mechanoradical initiator, and other components present in the reaction system are added to the reaction vessel is not particularly limited. Addition means is also not particularly limited. For example, all the components may be added to the reaction vessel at once, or after some of the components are added to the reaction vessel and reacted, the remaining components may be added to the reaction vessel.

The reaction product obtained after the mechanochemical treatment and the reaction using the polymer mechanoradical initiator may be purified, as needed. The purification method is not particularly limited, and for example, methods, such as filtration, distillation, recrystallization, column chromatography, and washing with a solvent, are used.

### <Component Used in Mechanochemical Treatment>

In the mechanochemical treatment, a component other than the substrate for the reaction using the organic polymer material and the polymer mechanoradical initiator can be used. For example, a solvent having a melting point of 30°C or lower can be used in an amount of 0.8 mL or less, preferably 0.5 mL or less, and more preferably 0.3 mL or less, based on a total of 1 mmol of the substrate in the reaction using the polymer mechanoradical initiator. The lower limit of the amount of the solvent having a melting point of 30°C or lower used may be 0 mL (no organic solvents are used). The amount of the organic solvent used in the mechanochemical treatment represents any of an embodiment in which the organic solvent is not used at all, an embodiment in which the solvent is not actively used, and an embodiment in which the organic solvent is used but is used only in such a small amount that the solvent effect is not provided.

Typically, when a reaction using a radical initiator is performed in a solution system, an organic solvent is used in an amount of 1 mL or more, preferably 10 mL or more, based on a total of 1 mmol of a substrate. In the present invention, the amount of the organic solvent used is 0.8 mL or less based on a total of 1 mmol of the substrate. Thus, components, such as the organic polymer material and the substrate for the reaction using the polymer mechanoradical initiator, are usually present at the start of the reaction in a state where at least a part thereof is not dissolved in the organic solvent or the like or where, in some cases, all of the components are not dissolved in the organic solvent or the like and are present in a solid state, and react.

Examples of the solvent that can be used in the mechanochemical treatment and that has a melting point of 30°C or lower include solvents used when reactions are performed in solution systems. Examples thereof include one or more types selected from the group consisting of oxygen-containing organic solvents, such as methanol, ethanol, n-propanol, isopropanol, 1-butanol, 1,1-dimethylethanol, tert-butanol, 2-methoxyethanol, ethylene glycol, diethyl ether, diisopropyl ether, dibutyl ether, t-butyl methyl ether, tetrahydrofuran, tetrahydropyran, cyclopentyl methyl ether, dimethoxyethane, 1,4-dioxane, anisole, acetoxy-2-ethoxyethane, propylene glycol monomethyl ether acetate, ethylene glycol dimethyl ether, diethylene glycol dimethyl ether, 1-methoxy-1,1,2,2-tetrafluoroethane, 1-ethoxy-1,1,2,2-tetrafluoroethane, acetone, methyl ethyl ketone, ethyl acetate, butyl acetate, and acetic acid; aromatic-based solvents, such as benzene, toluene, xylene, mesitylenedurene, and decalin; aliphatic-based organic solvents, such as hexane, pentane, and heptane; halogenated hydrocarbon-based organic solvents, such as dichloromethane, chloroform, carbon tetrachloride, chlorobenzene, and 1,2-dichlorobenzene; nitrogen-containing organic solvents, such as acetonitrile, N,N'-dimethylformamide, N,N'-dimethylacetamide, N-methyl-2-pyrrolidone, and pyridine; sulfur-containing organic solvents, such as dimethyl sulfoxide; and water and the like.

In the mechanochemical treatment, a solvent can be used as liquid assisted grinding (LAG). Among the above-described solvents, the liquid assisted grinding is preferably one or more types selected from the group consisting of, for example, oxygen-containing organic solvents, such as 2-methoxyethanol, ethylene glycol, diethyl ether, dibutyl ether, t-butyl methyl ether, tetrahydrofuran, 1,4-dioxane, propylene glycol monomethyl ether acetate, ethylene glycol dimethyl ether, diethylene glycol dimethyl ether, diethyl ether, dibutyl ether, and tetrahydrofuran; nitrogen-containing organic solvents, such as N',N'-dimethylformamide, N,N'-dimethylacetamide, N-methyl-2-pyrrolidone, and pyridine; and sulfur-containing organic solvents, such as dimethyl sulfoxide, and the like. Particularly preferably, one or more types selected from the group consisting of 2-methoxyethanol, ethylene glycol, diethyl ether, dibutyl ether, t-butyl methyl ether, tetrahydrofuran, N',N'-dimethylformamide, N,N'-dimethylacetamide, N-methyl-2-pyrrolidone, pyridine, and dimethyl sulfoxide can be used.

### [Polymer Mechanoradical Initiator]

In the present invention, the polymer mechanoradical initiator according to the first aspect of the invention is a polymer mechanoradical initiator obtained by the mechanochemical treatment of an organic polymer material, in which the polymer mechanoradical initiator is used for a reaction in which a polymer mechanoradical initiator component is not incorporated into a reaction product.

In the polymer mechanoradical initiator according to the first aspect of the invention, the details of the "organic polymer material" and the "mechanochemical treatment" are the same as those in [Organic Polymer Material] and [Mechanochemical Treatment] described above.

The polymer mechanoradical initiator is formed by mechanochemical treatment of the organic polymer material. The mechanical action of the mechanochemical treatment results in the homolytic cleavage of a polymer chain in the organic polymer material to form radicals at the cleaved portions of the polymer chain. The resulting radicals are generated as polymer mechanoradical initiators.

<Reaction in Which Polymer Mechanoradical Initiator Component Is not Incorporated into Reaction Product>

The polymer mechanoradical initiator according to the first aspect of the invention is used for a reaction in which a radical (free radical) is involved in the course of a chemical reaction and in which a polymer mechanoradical initiator component is not incorporated into a reaction product. The reaction in which the radical (free radical) is involved in the course of the chemical reaction is not particularly limited. In the reaction in which the polymer mechanoradical initiator component is not incorporated into the reaction product, the substrate is activated and reacted due to the transfer of the radical of the polymer mechanoradical initiator to the substrate, and the polymer mechanoradical initiator is deactivated due to the loss of the radical. Thus, the polymer constituting the polymer mechanoradical initiator does not react with the substrate to form a bond.

The reaction in which the polymer mechanoradical initiator component is not incorporated into the reaction product is not particularly limited as long as it is a reaction in which the polymer constituting the polymer mechanoradical initiator does not react with the substrate to form a bond. Examples thereof include a radical reduction reaction of an organohalogen compound, a radical cyclization reaction (intramolecular radical cyclization reaction) of an alkene compound or the like having a halogen group in its molecule, and a C-H fluorination reaction of a compound or the like having a C-H bond based on secondary carbon or tertiary carbon.

### [Reaction Method for Radical Reduction of Substrate Using Polymer Mechanoradical Initiator]

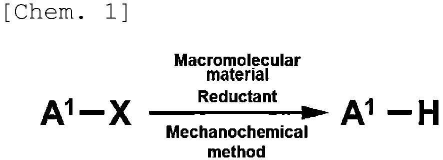

(In the formula, A¹ is an organic group, and X is Br, I, or Cl.)

In the present invention, the reaction method for the radical reduction of a substrate using a polymer mechanoradical initiator according to the second aspect of the invention is a reaction method for the reduction and dehalogenation of an organohalogen compound, which is a substrate, by subjecting an organic polymer material and the substrate to the mechanochemical treatment, in which a polymer mechanoradical initiator obtained by the mechanochemical treatment of the organic polymer material is used as a radical initiator.

In the reaction method for the radical reduction of the substrate using the polymer mechanoradical initiator according to the second aspect of the invention, the details of the "organic polymer material" and the "mechanochemical treatment" are the same as those in [Organic Polymer Material] and [Mechanochemical Treatment] described above, and the details of the "polymer mechanoradical initiator" are the same as those in [Polymer Mechanoradical Initiator] described above.

### <Organohalogen compound>

In the reaction method for the radical reduction of the substrate using the polymer mechanoradical initiator according to the second aspect of the invention, an example of the organohalogen compound serving as the substrate is a compound represented by formula (I):

A¹-Xₘ (I)

(where in the formula, A¹ represents any one of an m-valent aromatic hydrocarbon group that may have a substituent, an m-valent aromatic heterocyclic group that may have a substituent, an m-valent heterocyclic group that may have a substituent, an m-valent aliphatic hydrocarbon group that may have a substituent, and an m-valent unsaturated aliphatic hydrocarbon group that may have a substituent. X represents any one of chlorine, bromine, and iodine, and
when a plurality of X's are present, X's may be the same or different from each other. m is the number of X and represents an integer of 1 or more.).

One type of the organohalogen compound represented by formula (I) can be used, or two or more types can be used in combination. The organohalogen compound represented by formula (I) can be used as a commercially available product as it is or after purification.

### (A¹ Group in Formula (I))

The number of carbon atoms of the m-valent aromatic hydrocarbon group that may have a substituent, which serves as the A¹ group, is not particularly limited, and is, for example, 6 to 60, preferably 6 to 40, and more preferably 6 to 30.

In the m-valent aromatic hydrocarbon group, m is an integer of 1 or more, for example, 1 to 10, preferably 1 to 6, and more preferably 1 to 4.

In the m-valent aromatic hydrocarbon group that may have a substituent, which serves as the A¹ group, examples of the monovalent aromatic hydrocarbon group where m = 1 include a phenyl group, a naphthyl group, an anthracenyl group (or an anthracene group), a phenanthrenyl group (or a phenanthrene group), a biphenyl group, a terphenyl group, a pyrenyl group (or a pyrene group), a perylenyl group (or a perylene group), a triphenylenyl group (or a triphenylene group), a fluorenyl group, and a spirobifluorenyl group.

In the m-valent aromatic hydrocarbon group that may have a substituent, which serves as the A¹ group, an example of the m-valent aromatic hydrocarbon group where m is an integer of 2 or more is a group in which m - 1 hydrogen atoms are removed from the aromatic ring in each of the monovalent aromatic hydrocarbon groups described above.

The number of carbon atoms of the m-valent aromatic heterocyclic group that may have a substituent, which serves as the A¹ group, is not particularly limited, and is, for example, 4 to 60, preferably 4 to 40, and more preferably 4 to 30.

In the m-valent aromatic heterocyclic group that may have a substituent, which serves as the A¹ group, m is an integer of 1 or more, for example, 1 to 10, preferably 1 to 6, and more preferably 1 to 4.

In the m-valent aromatic heterocyclic group that may have a substituent, which serves as the A¹ group, examples of the monovalent aromatic heterocyclic group where m = 1 include sulfur-containing heteroaryl groups, such as a thiophenyl group (a thiophene group or a thienyl group), a benzothienyl group (a benzothiophene group), and a dibenzothienyl group (a dibenzothiophene group); oxygen-containing heteroaryl groups, such as a furanyl group (or a furan group), a benzofuranyl group (a benzofuran group), a dibenzofuranyl group (a dibenzofuran group), a phenyldibenzofuranyl group, and a dibenzofuranylphenyl group; nitrogen-containing heteroaryl groups, such as a pyridyl group (or a pyridine group), a pyrimidinyl group (or a pyrimidine group), a pyrazyl group (or a pyrazine group), a quinolyl group (a quinoline group), an isoquinolyl group (or an isoquinoline group), a carbazolyl group (or a carbazole group), a 9-phenylcarbazolyl group, an acridinyl group (or an acridine group), a quinazolyl group (or a quinazoline group), a quinoxalyl group (or a quinoxaline group), a 1,6-naphthyridinyl group, 1,8-naphthyridinyl group, and a porphyrin group (or a porphyrin ring); and heteroaryl groups each containing two or more types of hetero atoms (for example, nitrogen and sulfur), such as a benzothiazolyl group (or a benzothiazole group) and a benzothiadiazole group. Further examples thereof include a pyrrole group, a silole group, a borole group, a phosphole group, a selenophene group, a germole group, an indole group, an indene group, a benzosilole group, a benzoborole group, a benzophosphole group, a benzoselenophene group, a benzogermole group, a dibenzosilole group, a dibenzoborole group, a dibenzophosphole group, a dibenzoselenophene group, a dibenzogermole group, a dibenzothiophene 5-oxide group, a 9H-fluoren-9-one group, a dibenzothiophene 5,5-dioxide group, an azabenzothiophene group, an azabenzofuran group, an azaindole group, an azaindene group, an azabenzosilole group, an azabenzoborole group, an azabenzophosphole group, an azabenzoselenophene group, an azabenzogermole group, an azadibenzothiophene group, an azadibenzofuran group, an azacarbazole group, an azafluorene group, an azadibenzosilole group, an azadibenzoborole group, an azadibenzophosphole group, an azadibenzoselenophene group, an azadibenzogermole group, an azadibenzothiophene 5-oxide group, an aza-9H-fluoren-9-one group, an azadibenzothiophene 5,5-dioxide group, a pyridazine group, a triazine group, a phenanthroline group, a pyrazole group, an imidazole group, a triazole group, an oxazole group, an isoxazole group, a triazole group, an isothiazole group, an oxadiazole group, a thiadiazole group, a benzopyrazole group, a benzimidazole group, a benzoxazole group, a benzoxadiazole group, a 5,6,7,8-tetrahydroisoquinoline group, and a 5,6,7,8-tetrahydroquinoline group.

In the m-valent aromatic heterocyclic group that may have a substituent, which serves as the A¹ group, an example of the m-valent aromatic heterocyclic group where m is an integer of 2 or more is a group in which m - 1 hydrogen atoms are removed from the aromatic ring in each of the monovalent aromatic heterocyclic groups described above. Examples thereof include a benzo[1,2-c:4,5-c']bis[1,2,5]thiadiazole skeleton (a benzobisthiadiazole group), a thienylenyl group (or a thiophenediyl group), a phenyldibenzothienylenyl group, a dibenzothienylenylphenyl group, and a pyridylenyl group (or a pyridinediyl group).

The number of carbon atoms of the m-valent heterocyclic group that may have a substituent, which serves as the A¹ group, is not particularly limited, and is, for example, 0 to 60, preferably 0 to 40, and more preferably 0 to 30.

In the m-valent heterocyclic group, which serves as the A¹ group, m is an integer of 1 or more, for example, 1 to 10, preferably 1 to 6, and more preferably 1 to 4.

In the m-valent heterocyclic group that may have a substituent, which serves as the A¹ group, examples of the monovalent heterocyclic group where m = 1 include groups each having a structure in which one or more carbon atoms in a ring of an alicyclic hydrocarbon group, such as a cycloolefin group, are substituted with hetero atoms, such as nitrogen, oxygen, sulfur, and silicon. Examples thereof include a tetrahydrothienyl group, a tetrahydrofuranyl group, a pyrrolidinyl group, a piperazyl group, a morpholyl group, a monosaccharide group, and a disaccharide group.

In the m-valent heterocyclic group that may have a substituent, which serves as the A¹ group, an example of the m-valent heterocyclic group where m is an integer of 2 or more is a group in which m - 1 hydrogen atoms are removed from the monovalent heterocyclic group in each of the monovalent heterocyclic groups described above.

The number of carbon atoms of the m-valent aliphatic hydrocarbon group that may have a substituent, which serves as the A¹ group, is not particularly limited, and is, for example, 2 to 60, preferably 3 to 40, and more preferably 5 to 30.

In the m-valent aliphatic hydrocarbon group, which serves as the A¹ group, m is an integer of 1 or more, for example, 1 to 10, preferably 1 to 6, and more preferably 1 to 4.

In the m-valent aliphatic hydrocarbon group that may have a substituent, which serves as the A¹ group, examples of the monovalent aliphatic hydrocarbon group where m = 1 include saturated aliphatic hydrocarbon groups, such as an alkyl group and a cycloolefin group.

In the m-valent aliphatic hydrocarbon group that may have a substituent, which serves as the A¹ group, an example of the m-valent aliphatic hydrocarbon group where m is an integer of 2 or more is a group in which m - 1 hydrogen atoms are removed from each of the monovalent aliphatic hydrocarbon groups described above.

The m-valent aliphatic hydrocarbon group that may have a substituent, which serves as the A¹ group, may contain a heteroatom (for example, nitrogen, oxygen, phosphorus, or sulfur) in the main chain or substituent. Examples of the heteroatom-containing substituent include aromatic heterocyclic groups, such as a thiophenyl group, a furanyl group, and a pyrrole group, and saturated heterocyclic groups, such as a tetrahydrothienyl group, a tetrahydrofuranyl group, a pyrrolidinyl group, a piperazyl group, and a morpholyl group, which are described above.

The number of carbon atoms of the m-valent saturated aliphatic hydrocarbon group that may have a substituent, which serves as the A¹ group, is not particularly limited, and is, for example, 2 to 60, preferably 3 to 40, and more preferably 5 to 30.

In the m-valent unsaturated aliphatic hydrocarbon group, which serves as the A¹ group, m is an integer of 1 or more, for example, 1 to 10, preferably 1 to 6, and more preferably 1 to 4.

In the m-valent unsaturated aliphatic hydrocarbon group that may have a substituent, which serves as the A¹ group, examples of the monovalent unsaturated aliphatic hydrocarbon group where m = 1 include an alkenyl group and an alkynyl group.

In the m-valent unsaturated aliphatic hydrocarbon group that may have a substituent, which serves as the A¹ group, an example of the m-valent unsaturated aliphatic hydrocarbon group where m is an integer of 2 or more is a group in which m - 1 hydrogen atoms are removed from each of the monovalent unsaturated aliphatic hydrocarbon groups described above.

The m-valent unsaturated aliphatic hydrocarbon group that may have a substituent, which serves as the A¹ group, may contain a heteroatom (for example, nitrogen, oxygen, phosphorus, or sulfur) in the main chain or substituent. Examples of the heteroatom-containing substituent include aromatic heterocyclic groups, such as a thiophenyl group, a furanyl group, and a pyrrole group, and saturated heterocyclic groups, such as a tetrahydrothienyl group, a tetrahydrofuranyl group, a pyrrolidinyl group, a piperazyl group, and a morpholyl group, which are described above.

In the organohalogen compound represented by formula (I), as A¹ in formula (I), for example, the following groups can be exemplified:
phenyl groups, (such as an alkyl (for example, methyl or the like) phenyl group, a dialkyl (for example, dimethyl) phenyl group, an alkoxy (for example, methoxy or the like) phenyl group, a dialkylamino (for example, dimethylamino or the like) phenyl group, a diaryl (for example, diphenyl or the like) aminophenyl group, a perfluoroalkyl (for example, trifluoromethyl or the like) phenyl group, an alkyl (for example, ethyl or the like) oxycarbonylphenyl group, an alkanoyl group (for example, (acyl)phenyl or the like), and a phenyl group linked by a linking group, for example an alkylene group, an ether group, or an ester group;
naphthyl groups, such as a naphthyl group, an aryl (for example, phenyl or the like) naphthyl group, a naphthyl group having an alkylene (for example, ethylene or the like) linkage, and a naphthyl group having an arylene (for example, phenylene or the like) linkage;
a phenanthrenyl group;
anthracenyl groups, such as an anthracenyl group, an aryl (for example, phenyl or the like) anthracenyl group, a diaryl (for example, dinaphthyl or the like) anthracenyl group, and a diarylboryl (for example, bis(trialkylphenyl)boryl or the like) anthracenyl group;
pyrenyl groups, such as a pyrenyl group and an alkyl (for example, tert-butyl or the like) pyrenyl group;
biphenyl groups, such as a biphenyl group and a biphenyl group having an alkylene (for example, propylene, isopropylene, or the like) linkage;
terphenyl groups, such as a terphenyl group and a tetraaryl (for example, tetraphenyl or the like) terphenyl group;
a triphenylenyl group;
a fluorenyl group and a spirobifluorenyl group;
a 2-aryl (for example, phenyl or the like) ethenylphenyl group, a 1,2,2-triaryl (for example, triphenyl or the like) ethenylphenyl group, and a 2-aryl (for example, phenyl or the like) ethenylphenyl group;
an aryl (for example, phenyl or the like)-substituted carbazolyl group;
an anthracene-9,10-dione group;
an aryl (for example, phenyl or the like)-substituted thienyl group, a thiophene group, and a benzothiadiazole group; and
divalent or higher-valent groups, such as a phenylene group, an aryl (for example, bis(3,5-methylphenyl) or the like) porphyrin ring, a pyrene-tetra-yl group, and a benzo[1,2-c:4,5-c']bis[1,2,5]thiadiazole skeleton (a benzobisthiadiazole group).

The substituent of the m-valent aromatic hydrocarbon group that may have a substituent, the m-valent aromatic heterocyclic group that may have a substituent, the m-valent aliphatic hydrocarbon group that may have a substituent, or the m-valent unsaturated aliphatic hydrocarbon group that may have a substituent, which serves as the A¹ group, is not particularly limited as long as a reaction targeted by the present invention can be performed.

Examples of the substituent include one or more types selected from the group consisting of alkyl groups each having 1 to 24, preferably 1 to 18, more preferably 1 to 12, and still more preferably 1 to 8 carbon atoms (such as a methyl group, an ethyl group, a n-propyl group, an isopropyl group, a n-butyl group, an isobutyl group, a tert-butyl group, a pentyl group, a hexyl group, an octyl group, and the like); alkoxy groups each having 1 to 24, preferably 1 to 18, more preferably 1 to 12, still more preferably 1 to 8 carbon atoms (such as a methoxy group, an ethoxy group, a n-propoxy group, an isopropoxy group, a n-butoxy group, an isobutoxy group, a tert-butoxy group, a pentyloxy group, a hexyloxy group, an octyloxy group, and the like); cycloalkyl groups each having 3 to 24, preferably 3 to 18, more preferably 3 to 12, still more preferably 3 to 8 carbon atoms (such as a cyclopropyloxy group, a cyclobutyloxy group, a cyclopentyloxy group, a cyclohexyloxy group, and the like); alkenyl groups each having 1 to 24, preferably 1 to 18, more preferably 1 to 12, still more preferably 1 to 8 carbon atoms (such as an ethenyl group, a propenyl group, a butenyl group, a pentenyl group, a hexenyl group, an octenyl group, and the like); alkynyl groups each having 1 to 24, preferably 1 to 18, more preferably 1 to 12, still more preferably 1 to 8 carbon atoms (such as an ethynyl group, a propynyl group, a butynyl group, a pentynyl group, a hexynyl group, an octynyl group, and the like); aryl groups each having 5 to 24, preferably 5 to 18, more preferably 5 to 12, still more preferably 5 to 8 carbon atoms (such as a phenyl group, a naphthyl group, a biphenyl group, and the like); arylalkyl groups each having 7 to 24, preferably 7 to 19, more preferably 7 to 13, still more preferably 7 to 9 carbon atoms (such as a monophenylmethyl group, a monophenylpropyl group, a triphenylmethyl group, and the like); aryloxy groups each having 5 to 24, preferably 5 to 18, more preferably 5 to 12, still more preferably 5 to 8 carbon atoms (such as a phenoxy group, a naphthyloxy group, a biphenyloxy group, and the like); heteroaryl groups each having 4 to 24, preferably 4 to 18, more preferably 4 to 12, still more preferably 4 to 8 carbon atoms (such as a thiophenyl group, a furanyl group, a carbazole group, a benzothiophenyl group, a benzofuranyl group, an indolyl group, a pyrrolyl group, a pyridyl group, and the like); acyl groups each having 1 to 24, preferably 1 to 18, more preferably 1 to 12, still more preferably 1 to 8 carbon atoms (such as an acetyl group, a propionyl group, a butanoyl group, a pentanoyl group, a heptanoyl group, a group in which a carbonyl group contained in each of the acyl groups is substituted with an ester group or an amide group, and the like; amino groups each having 1 to 24, preferably 1 to 18, more preferably 1 to 12, still more preferably 1 to 8 carbon atoms (such as a diphenylamino group, a dimethylamino group, and the like); fluorine, and fluorine-containing groups, such as fluorine-containing hydrocarbon groups each having 1 to 30, preferably 1 to 12 carbon atoms; and a cyano group, a nitro group, and the like.

The substituents may be cross-linked to each other, or the substituents may form a cyclic structure (aromatic group) together. The substituent may further have a substituent.

### (X Group in Formula (I))

The X group represents any one of chlorine, bromine, and iodine. When a plurality of X's are present, X's may be the same or different. Depending on the reactivity and the like, an appropriate group can be used.

The number of the X groups in formula (I), m, is an integer of 1 or more, and is not particularly limited as long as it is within a range in which a reaction targeted by the present invention can be performed. For example, m can be 10 or less, preferably 8 or less, more preferably 6 or less, and still more preferably 4 or less.

The organohalogen compound may be waste. Waste organohalogen compounds can be rendered harmless and nontoxic through radical reduction reactions. Examples of waste organohalogen compounds include flame retardants, agricultural chemicals, and cleaning agents. These can be dehalogenated and rendered harmless.

### <Reducing Agent>

In the reaction method for the radical reduction of a substrate using the polymer mechanoradical initiator according to the second aspect of the invention, examples of the reducing agent that can be used as needed include known radical reducing agents. Examples thereof include one or more types selected from the group consisting of silane-based reducing agents, tin-based reducing agents, and hypophosphorous acid-based reducing agents.

Examples of the silane-based reducing agent include one or more types of compounds represented by formula (II):

(R¹)_{4-p-q}SiHₚ(SH)_{q} (II)

(where in the formula, R¹ is a monovalent organic group, and when a plurality of R¹ groups are present, they may be the same or different, and two or more R¹ groups may be bonded to form a ring. p is an integer of 0 to 4, q is an integer of 0 to 3, and p + q is an integer of 1 to 4).

For example, R¹ is preferably an optionally substituted linear or branched alkyl group having 1 to 12 carbon atoms, an optionally substituted aromatic group having 6 to 20 carbon atoms, or an optionally substituted silyl group.

When q ≠ 0, preferably, p + q ≤ 3. In addition, when q ≠ 0, at least one R¹ is preferably an optionally substituted silyl group.

Examples of the tin-based reducing agent include one or more types of compounds represented by formula (III):

(R²)₄₋ᵣSnHᵣ (III)

(wherein in the formula, R² is a monovalent organic group, and when a plurality of R² groups are present, they may be the same or different, and two or more R² groups may be bonded to form a ring. r is an integer of 1 to 4.).

For example, R² is preferably an optionally substituted linear or branched alkyl group having 1 to 12 carbon atoms, or an optionally substituted aromatic group having 6 to 20 carbon atoms.

Examples of the hypophosphorous acid-based reducing agent include hypophosphorous acid (H₃PO₂) or one or more types of salts thereof. Examples of the salts of hypophosphorous acid include one or more types selected from the group consisting of inorganic salts and salts of organic bases, such as amine-based compounds.

Specific examples of the reducing agent include one or more types of compounds below. Note that "i-Pr" stands for an isopropyl group, and "n-Bu" stands for a normal butyl group.

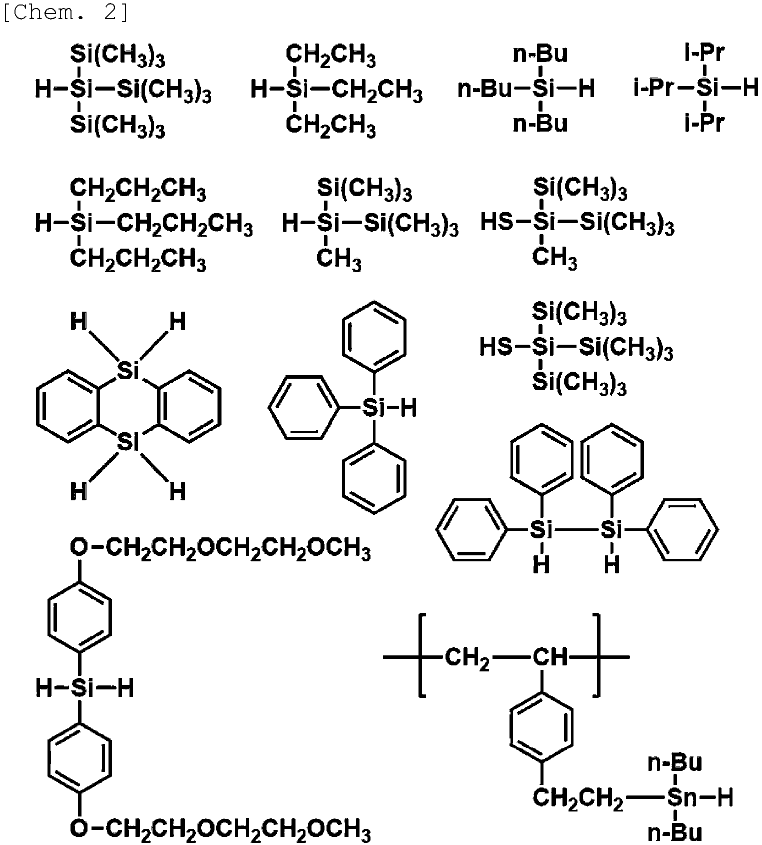

**H-Sn(CH₂CH₂C₆F₁₃)₃ H₃PO₂/N(CH₂CH₃)₃**

**H-Sn(CH₂CH₃)₃ H-Sn(n-Bu)₃ H₃PO₂**/**N(n-Bu)₃**

In the reaction method for the radical reduction of a substrate using the polymer mechanoradical initiator according to the second aspect of the invention, the amount of the reducing agent used is not particularly limited. For example, the amount is 5.0 equivalents or less, preferably 3.0 equivalents or less, and more preferably 2.5 equivalents or less, based on 1 equivalent of the substrate.

### <Chain Transfer Agent>

In the reaction method for the radical reduction of a substrate using the polymer mechanoradical initiator according to the second aspect of the invention, examples of the chain transfer agent that is used as needed include known chain transfer agents. Examples thereof include one or more types selected from the group consisting of a thiol-based chain transfer agent, a thiocarbonylthio-based chain transfer agent, an aromatic α-methylalkenyl-based chain transfer agent, and an amine-based chain transfer agent.

Examples of the thiol-based chain transfer agent include compounds having one or more thiol groups. Examples thereof include one or more types selected from the group consisting of 1-decanethiol, 1-dodecanethiol, 2-ethylhexyl mercaptoacetate, 2-mercaptoethanol, 3-mercapto-1,2-propanediol, 3-mercaptopropionic acid, methyl mercaptoacetate, mercaptosuccinic acid, thiophenol, 3-tridecyl mercaptopropionate, 2-mercaptobenzothiazole, 2-mercaptobenzoimidazole, 2-mercaptobenzoxazole, 3-mercapto-1,2,4-triazole, octyl thioglycolate, octyl 2-mercaptopropionate, octyl 3-mercaptopropionate, mercaptopropionic acid 2-ethylhexyl ester, octanoic acid 2-mercaptoethyl ester, 1,4-dimethylmercaptobenzene, 1,3,5-triazine-2,4,6-trithiol, 1,8-dimercapto-3,6-dioxaoctane, ethylene glycol bisthioglycolate, trimethylolpropane tristhioglycolate, trimethylolpropane tristhiopropionate, trimethylolpropane tristhiobutanate, pentaerythritol tetrakisthioglycolate, pentaerythritol tetrakisthiopropionate, pentaerythritol tetrakis(4-mercaptobutanate), pentaerythritol tetrakis(6-mercaptohexanoate), dipentaerythritol hexakis(3-mercaptopropionate), trimethylolpropane tris(3-mercaptopropionate), trimethylolpropane tris(3-mercaptobutyrate), pentaerythritol tetrakis(3-mercaptobutyrate), pentaerythritol tetrakis(3-mercaptopropionate, dipentaerythritol hexakis(3-mercaptobutyrate), 1,3,5-tris[2-(3-mercaptobutyryloxy)ethyl]-1,3,5-triazine-2,4,6(1H,3H,5H)-trione, tris[(3-mercaptopropionyloxy)-ethyl]-isocyanurate, 1,4-bis(3-mercaptobutyryloxy)butane, 1,2-ethanedithiol, 1,3-propanedithiol, 1,4-butanedithiol, 1,6-hexanedithiol, 1,8-octanedithiol, 1,2-cyclohexanedithiol, decanedithiol, ethylene glycol bisthiopropionelate, ethylene glycol bisthioglycolate, 1,4-butanediol bisthiopropionate, trimercaptopropionate tris(2-hydroxyethyl) isocyanurate, 1,4-dimethylmercaptobenzene, 2-(N,N-dibutylamino)-4,6-dimercapto-s-triazine, and the like.

Examples of the thiocarbonylthio-based chain transfer agent include one or more types selected from the group consisting of benzyl dithiobenzoate, 1-acetoxyethyl dithiobenzoate, 1,4-bis(thiobenzoylthiomethyl)benzene, benzyl dithioacetate, t-butyl trithiobenzoate, carboxymethyl dithiobenzoate, 2-phenyl-2-propyl benzodithioate, 1-(methoxycarbonyl)ethyl benzodithioate, ethyl-2-methyl-2-(phenylthiocarbonylthio)propionate, methyl-2-phenyl-2-(phenylcarbonothioylthio)acetate, ethyl-2-(phenylcarbonothioylthio)propionate, bis(thiobenzoyl) disulfide, 2-(dodecylthiocarbonylthioylthio)propionic acid, 2-(dodecylthiocarbonylthioylthio)-2-methylpropionic acid, methyl-2-(dodecylthiocarbonylthioylthio)-2-methylpropionate, 2-(dodecylthiocarbonylthioylthio)-2-methylpropionic acid N-hydroxysuccinimide ester, poly(ethylene glycol) methyl ether (2-methyl-2-propionic acid dodecyl trithiocarbonate), poly(ethylene glycol) bis[2-(dodecylthiocarbonylthioylthio)-2-methylpropionate], 2-(dodecylthiocarbonylthioylthio)-2-methyl propionic acid 3-azido-1-propanol ester, 2-(dodecylthiocarbonylthioylthio)-2-methyl propionic acid pentafluorophenyl ester, bis(dodecylsulfanylthiocarbonyl) disulfide, 4-cyano-4-(phenylcarbonothioylthio)pentanoic acid, 2-cyano-2-propylbenzodithioate, 4-cyano-4-[(dodecylsulfanylthiocarbonyl)sulfanyl]pentanoic acid, 2-cyano-2-propyl dodecyl trithiocarbonate, cyanomethyl-N-methyl-N-phenyldithiocarbamate, S-cyanomethyl-S-dodecyl trithiocarbonate, 2-cyanopropan-2-yl benzothioate, 4-cyano-4-(thiobenzoylthio)valeric acid, 2-cyano-2-propyl dodecyl trithiocacarbonate, 4-cyano-4-[(dodecylsulfanylthiocarbonyl)sulfanyl]valeric acid, 2-(dodecylthiocarbonothioylthio)-2-methylpropionic acid, 2-cyano-2-propyl 4-cyanobenzodithioate, 4-cyano-4-(phenylcarbonothioylthio)pentanoic acid, 4-cyano-4-(phenylcarbonothioylthio)pentanoic acid N-succinimidyl ester, 4-cyano-4-[(dodecylsulfanylthiocarbonyl)sulfanyl]pentanol, poly(ethylene glycol) methyl ether 4-cyano-4-[(dodecylsulfanylthiocarbonyl)sulfanyl]pentanoate, poly(ethylene glycol) methyl ether (4-cyano-4-pentanoate dodecyl trithiocarbonate), poly(ethylene glycol) methyl ether (4-cyano-4-pentanoate dodecyl trithiocarbonate), poly(ethylene glycol) methyl ether (4-cyano-4-pentanoate dodecyl trithiocarbonate), cyanomethyl dodecyl trithiocarbonate, cyanomethyl methyl(phenyl)carbamodithioate, cyanomethyl diphenylcarbamodithioate, 1-succinimidyl-4-cyano-4-[N-methyl-N-(4-pyridyl)carbamothioylthio]pentanoate, 2-cyanopropan-2-yl N-methyl-N(pyridin-4-yl)carbamodithioate, cyanomethyl methyl(4-pyridyl)carbamodithioate, and the like.

Examples of the aromatic α-methylalkenyl-based chain transfer agent include 2,4-diphenyl-4-methyl-1-pentene and the like.

Examples of the amine-based chain transfer agent include one or more types selected from the group consisting of triethylamine, p-dimethylaminobenzene carboxylic acid ethyl ester, N-phenylglycine, 3-amino-4-methoxybenzenesulfonylbenzyl, 3-amino-4-methoxybenzenesulfonyldiethylamine, triethylamine, butylamine, and the like.

In the reaction method for the radical reduction of a substrate using the polymer mechanoradical initiator according to the second aspect of the invention, the amount of the chain transfer agent used is not particularly limited. For example, the amount is 5.0 equivalents or less, preferably 3.0 equivalents or less, and more preferably 2.5 equivalents or less, based on 1 equivalent of the substrate.

### <Other Components>

In the reaction method for the radical reduction of a substrate using the polymer mechanoradical initiator according to the second aspect of the invention, other components can be further used in addition to the "organic polymer material," "substrate," "reducing agent," "chain transfer agent," "solvent having a melting point of 30°C or lower," and "liquid assisted grinding". As the other components, for example, a reaction promoter (for example, water), a base, and the like may be used.

### [Reaction Method for Radical Cyclization of Substrate Using Polymer Mechanoradical Initiator]

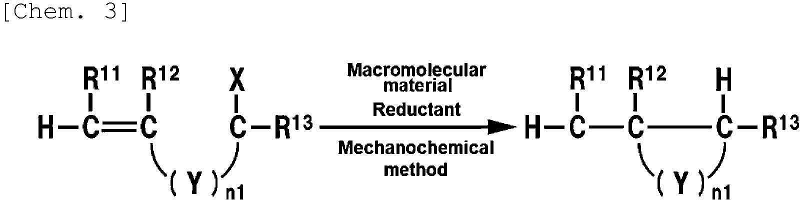

(In the formula, each Y is any one of -CR¹⁴R¹⁵-, -O-, -NR¹⁶-, and -S-, and a plurality of Y's may be the same or different. R¹¹, R¹², R¹³, R¹⁴, R¹⁵, and R¹⁶ are monovalent organic groups that may be the same or different from one another. X is Br, I, or Cl, and n1 is an integer of 1 to 6.)

In the present invention, the reaction method for the radical cyclization (intramolecular radical cyclization) of a substrate using the polymer mechanoradical initiator according to the third aspect of the invention is a reaction method for the intramolecular radical cyclization of an organohalogen compound, which is a substrate having a specific structure in its molecule, by subjecting an organic polymer material and the substrate to the mechanochemical treatment, in which the polymer mechanoradical initiator obtained by the mechanochemical treatment of the organic polymer material is used as a radical initiator.

In the reaction method for the radical cyclization (intramolecular radical cyclization) of the substrate using the polymer mechanoradical initiator according to the third aspect of the invention, the details of the "organic polymer material" and the "mechanochemical treatment" are the same as those in [Organic Polymer Material] and [Mechanochemical Treatment] described above, and the details of the "polymer mechanoradical initiator" are the same as those in [Polymer Mechanoradical Initiator] described above.

As the substrate in the reaction method for the radical cyclization (intramolecular radical cyclization) of the substrate using the polymer mechanoradical initiator according to the third aspect of the invention, among the organohalogen compounds serving as the substrates in the radical reduction reaction method used in the "Reaction Method for Radical Reduction of Substrate Using Polymer Mechanoradical Initiator" according to the second aspect of the invention, a compound represented by formula (IV) is exemplified: 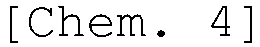 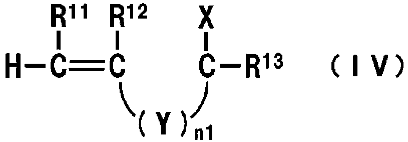 (where in the formula, each Y is any one of -CR¹⁴R¹⁵-, -O-, - NR¹⁶-, and -S-, and a plurality of Y's may be the same or different. R¹¹, R¹², R¹³, R¹⁴, R¹⁵, and R¹⁶ are monovalent organic groups that may be the same or different. X is Br, I, or Cl, and n1 is an integer of 1 to 6.).

### [Reaction Method for C-H Fluorination of Substrate Using Polymer Mechanoradical Initiator]

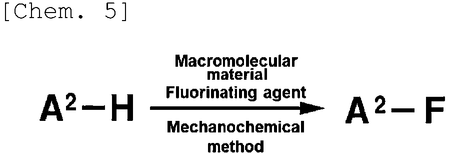

(In the formula, A² represents any one of an m-valent aromatic hydrocarbon group that may have a substituent, an m-valent aromatic heterocyclic group that may have a substituent, an m-valent heterocyclic group that may have a substituent, an m-valent aliphatic hydrocarbon group that may have a substituent, and an m-valent unsaturated aliphatic hydrocarbon group that may have a substituent.)

In the present invention, the reaction method for the C-H fluorination (C-H radical fluorination) of a substrate using the polymer mechanoradical initiator according to a fourth aspect of the invention is a reaction method for the C-H fluorination of a compound having a C-H bond based on secondary carbon or tertiary carbon, which serves as a substrate, by subjecting an organic polymer material, the substrate, and a fluorinating agent to the mechanochemical treatment, in which the polymer mechanoradical initiator obtained by the mechanochemical treatment of the organic polymer material is used as a radical initiator.

In the reaction method for the C-H fluorination (C-H radical fluorination) of the substrate using the polymer mechanoradical initiator according to the fourth aspect of the invention, the details of the "organic polymer material" and the "mechanochemical treatment" are the same as those in [Organic Polymer Material] and [Mechanochemical Treatment] described above, and the details of the "polymer mechanoradical initiator" are the same as those in [Polymer Mechanoradical Initiator] described above.

The substrate in the reaction method for the C-H fluorination (C-H radical fluorination) of the substrate using the polymer mechanoradical initiator according to the fourth aspect of the invention is represented by formula (V) :

A²-H (V)

(where in the formula, A² represents any one of an m-valent aromatic hydrocarbon group that may have a substituent, an m-valent aromatic heterocyclic group that may have a substituent, an m-valent heterocyclic group that may have a substituent, an m-valent aliphatic hydrocarbon group that may have a substituent, and an m-valent unsaturated aliphatic hydrocarbon group that may have a substituent.), and a compound having a C-H bond based on secondary carbon or tertiary carbon is exemplified.

The A² group in formula (V) can be the same group as the A¹ group in the above (the A¹ group in formula (I)).

The compound having a C-H bond based on secondary carbon is a compound having, in its molecule, a structure represented by formula (VI).

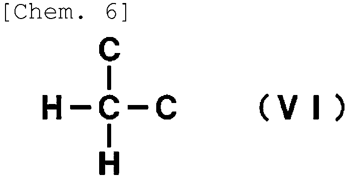

The compound having a C-H bond based on tertiary carbon is a compound having, in its molecule, a structure represented by formula (VII).

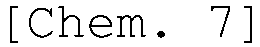

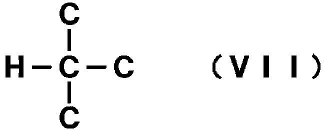

### <Fluorinating Agent>

The fluorinating agent in the reaction method for the C-H fluorination (C-H radical fluorination) of the substrate using the polymer mechanoradical initiator according to the fourth aspect of the invention is not particularly limited as long as it is a fluorinating agent that can fluorinate a C-H bond based on secondary carbon or tertiary carbon. Examples thereof include electrophilic fluorinating agents. The fluorinating agent used may be a commercially available product.

Examples of the fluorinating agent include one or more types selected from the group consisting of 1-chloromethyl-4-fluoro-1,4-diazoniabicyclo[2.2.2]octane bis(tetrafluoroborate) (Selectfluor (registered trademark)), 1-fluoro-4-hydroxy-1,4-diazadicyclo[2.2.2.]octane-di-tetrafluoroborate (Accufluor (registered trademark) NFTh), 1-fluoro-benzenesulfoneamide (Accufluor (registered trademark) NFSi), XeF₂, 1-(chloromethyl)-4-fluoro-1,4-diazoniabicyclo[2.2.2]octane bis(hexafluorophosphate), 1-(chloromethyl)-4-fluoro-1,4-diazoniabicyclo[2.2.2]octane bis(triflate), 1-fluoro-2,6-dichloropyridimium tetrafluoroborate, 1-fluoro-2,6-dichloropyridimium triflate, 1-fluoropyridinium pyridine heptafluoroborate, 1-fluoropyridinium tetrafluoroborate, 1-fluoropyridinium triflate, 1-fluoro-2,4,6-trimethylpyridinium tetrafluoroborate, 1-fluoro-2,4,6-trimethylpyridinium triflate, N,N'-difluoro-2,2'-bipyridinium bis(tetrafluoroborate), 1,1,2,3,3,3-hexafluoro-1-diethylaminopropane, triethylamine trihydrofluoride, diethylaminosulfur trifluoride, IF₅-pyridine-HF, IF₅-triethylamine-3HF, and the like.

Among these, one or more types selected from the group consisting of 1-chloromethyl-4-fluoro-1,4-diazoniabicyclo[2.2.2]octane bis(tetrafluoroborate) (Selectfluor (registered trademark)), 1-fluoro-4-hydroxy-1,4-diazadicyclo[2.2.2.]octane-di-tetrafluoroborate (Accufluor (registered trademark) NFTh), 1-fluoro-benzenesulfoneamide (Accufluor (registered trademark) NFSi) are preferred.

### EXAMPLES

The present invention will be described in detail below with reference to specific examples. These specific examples are merely illustrative of one aspect of the present invention. The present invention is not limited to these examples in any way.

The term "equiv" stands for "equivalent".

In each of examples and comparative examples, when a reaction was performed with a ball mill, reagents were added to a stainless steel ball mill jar, and a ball mill MM400 manufactured by Verder Scientific Co., Ltd. (formerly Retsch) was used.

When the reaction targeted by the present invention was performed with the ball mill, the outside of the ball mill jar was heated to a predetermined temperature with a heat gun (HG-1450B, manufactured by Takagi Co., Ltd.).

The relationship between the set temperature of the heat gun and the internal temperature of the ball mill jar (the temperature of the reaction system) was examined using a thermographic image. The following results were obtained.

**[Table 1]**

| Heat gun temperature | Temperature inside jar |
|---|---|
| 300°C | 125°C |
| 250°C | 120°C |
| 200°C | 100°C |
| 160°C | 80°C |
| 150°C | 74°C |
| 100°C | 60°C |
| No Heating | 35°C |

### [Radical Reduction Reaction of Organohalogen Compound]

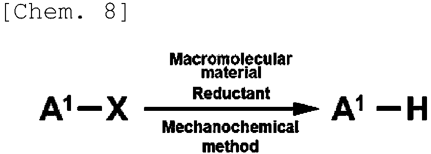

### {Study of Organic Polymer Material}

### <Example 1>

To a 5-mL stainless steel ball mill jar containing one stainless steel ball having a diameter of 10 mm, 44.2 mg (0.2 mmol) of 1-bromodecane (A-1) as a substrate, 59.7 mg (0.24 mmol) of tris(trimethylsilyl)silane as a reducing agent, and 200 mg of an ultra-high-molecular-weight polyethylene ("434272-100G" manufactured by Aldrich) (B-1) as an organic polymer material were added under air. The lid of the ball mill jar was closed. Thereafter, the ball mill jar was attached to a ball mill. The reaction was performed by shaking and stirring at room temperature (internal temperature: 35°C) for 60 minutes at a vibration frequency of 30 Hz. After the reaction was completed, the mixture was dissolved in ethyl acetate and filtered. The ethyl acetate was then removed using an evaporator. The crude product was purified by silica gel column chromatography to provide n-decane (C-1) as the reaction product. The gas chromatograph yield (GC yield) of the reaction product was 29%.

### <Example 2>

The reaction product (C-1) was obtained in the same manner as in Example 1, except that the ball mill jar was attached to the ball mill, and the reaction was performed by shaking and stirring at a vibration frequency of 30 Hz for 60 minutes while the ball mill jar was heated from the outside with the heat gun set at 100°C (internal temperature: 60°C). The GC yield of the reaction product was 82%.

### <Example 3>

The reaction product (C-1) was obtained in the same manner as in Example 1, except that 1-bromodecane (A-1), tris(trimethylsilyl)silane, and an ultra-high-molecular-weight polyethylene (B-1) were added to a ball mill jar under an Ar atmosphere, and the reaction was performed with the lid of the ball mill jar closed. The GC yield of the reaction product was 81%.

From the results of Examples 1 and 2, which were performed in the air atmosphere and Example 3, which was performed in the Ar atmosphere (inert atmosphere) under the same conditions except for the atmosphere, it can be said that the polymer radical initiator of the present invention is a polymer radical initiator that is less likely to be affected by oxygen, that is stable, and that has excellent reactivity.

### <Comparative Example 1>

The reaction was performed as in Example 1, except that the ultra-high-molecular-weight polyethylene was not added to the ball mill jar (the reaction was performed without any polymer material). The GC yield of the reaction product (C-1) was less than 1%.

### <Comparative Example 2>

The reaction was performed as in Example 1, except that 1-bromodecane (A-1), tris(trimethylsilyl)silane, and the ultra-high-molecular-weight polyethylene (B-1) were added to a test tube containing a magnetic stirrer and reacted under stirring. The GC yield of the reaction product (C-1) was less than 1%.

### <Example 4>

The reaction product (C-1) was obtained in the same manner as in Example 1, except that polyvinyl acetate (Mn = 3.5 kg/mol, Mw = 8.2 kg/mol (measured by SEC)) (B-2) was used as the organic polymer material instead of the ultra-high-molecular-weight polyethylene (B-1). The GC yield of the reaction product was 75%.

### <Example 5>

The reaction product (C-1) was obtained in the same manner as in Example 1, except that polypropylene (Mn = 67 kg/mol (catalog value)) (B-3) was used as the organic polymer material instead of the ultra-high-molecular-weight polyethylene (B-1). The GC yield of the reaction product was 10%.

### <Example 6>

The reaction product (C-1) was obtained in the same manner as in Example 1, except that polymethyl methacrylate (Mw = 120 kg/mol (catalog value)) (B-4) was used as the organic polymer material instead of the ultra-high molecular weight polyethylene (B-1). The GC yield of the reaction product was 4%.

### <Example 7>

The reaction product (C-1) was obtained in the same manner as in Example 1, except that polystyrene (Mw = 400 kg/mol (catalog value)) (B-5) was used as the organic polymer material instead of the ultra-high-molecular-weight polyethylene (B-1). The GC yield of the reaction product was 2%.

### {Study of Reaction Substrate}

### <Example 8>

To a 5-mL stainless steel ball mill jar containing a stainless steel ball having a diameter of 10 mm, 44.2 mg (0.2 mmol) of 1-bromodecane (A-1) as a substrate, 119.3 mg (0.48 mmol (2.4 equiv based on the substrate)) of tris(trimethylsilyl)silane as a reducing agent, and 300 mg of an ultra-high-molecular-weight polyethylene ("434272-100G" manufactured by Aldrich) (B-1) as an organic polymer material were added under air. Thereafter, the lid of the ball mill jar was closed. The ball mill jar was attached to a ball mill. The reaction was performed for 60 minutes by shaking and stirring at a vibration frequency of 30 Hz while the ball mill jar was heated from the outside (internal temperature: 100°C) with a heat gun set at 200°C. After the reaction was completed, the mixture was dissolved in ethyl acetate and filtered. The ethyl acetate was then removed using an evaporator. The crude product was purified by silica gel column chromatography to provide n-decane (C-1) as the reaction product. The GC yield of the reaction product was 82%.

### <Examples 9 to 19>

Reaction products (C-1) to (C-6) given in Table 2 were obtained in the same manner as in Example 8, except that compounds (A-2) to (A-12) given in Table 2 were used as the substrates. The GC yields of the reaction products are also presented in Table 2.

### <Examples 20 to 26>

Reaction products (C-7) to (C-13) given in Table 2 were obtained in the same manner as in Example 8, except that compounds (A-13) to (A-19) given in Table 2 were used as the substrates. The isolated yields of the reaction products are also presented in Table 2.

**[Table 2]**

| Example | Substrate | Reaction product | Yield |
|---|---|---|---|
| 8 | A-1 | C-1 | 82% (GC yield) |
| 9 | A-2 | C-1 | 69% (GC yield) |
| 10 | A-3 | C-1 | 5% (GC yield) |
| 11 | A-4 | C-2 | 67% (GC yield) |
| 12 | A-5 | C-2 | 87% (GC yield) |
| 13 | A-6 | C-2 | 76% (GC yield) |
| 14 | A-7 | C-2 | 8% (GC yield) |
| 15 | A-8 | C-3 | 61% (GC yield) |
| 16 | A-9 | C-4 | 72% (GC yield) |
| 17 | A-10 | C-5 | 81% (GC yield) |
| 18 | A-11 | C-4 | 54% (GC yield) |
| 19 | A-12 | C-6 | 68% (GC yield) |
| 20 | A-13 | C-7 | 99% (Isolated yield) |
| 21 | A-14 | C-8 | 80% (Isolated yield) |
| 22 | A-15 | C-9 | 71% (Isolated yield) |
| 23 | A-16 | C-10 | 95% (Isolated yield) |
| 24 | A-17 | C-11 | 80% (Isolated yield) |
| 25 | A-18 | C-12 | 80% (Isolated yield) |
| 26 | A-19 | C-13 | 87% (Isolated yield) |

### <Example 27>

To a 5-mL stainless steel ball mill jar containing a stainless steel ball having a diameter of 10 mm, 44.2 mg (0.2 mmol) of 1-bromodecane (A-1) as a substrate, 59.7 mg (0.24 mmol (1.2 equiv based on the substrate)) of tris(trimethylsilyl)silane as a reducing agent, and 200 mg of polyvinyl acetate (Mn = 3.5 kg/mol, Mw = 8.2 kg/mol (measured by SEC) (B-2)) as an organic polymer material were added under air. The lid of the ball mill jar was closed. Thereafter, the ball mill jar was attached to a ball mill. The reaction was performed by shaking and stirring at room temperature (internal temperature: 35°C) for 60 minutes at a vibration frequency of 30 Hz. After the reaction was completed, the mixture was dissolved in ethyl acetate and filtered. The ethyl acetate was then removed using an evaporator. The crude product was purified by silica gel column chromatography to provide n-decane (C-1) as the reaction product. The GC yield of the reaction product was 76%.

### <Examples 28 to 35>

The reaction products (C-1), (C-2), (C-4), and (C-5) given in Table 3 were obtained in the same manner as in Example 27, except that the compounds (A-2) to (A-6) and (A-9) to (A-11) given in Table 3 were used as the substrates. The GC yields of the reaction products are also presented in Table 3.

**[Table 3]**

| Example | Substrate | Reaction product | Yield |
|---|---|---|---|
| 27 | A-1 | C-1 | 76% (GC yield) |
| 28 | A-2 | C-1 | 63% (GC yield) |
| 29 | A-3 | C-1 | 12% (GC yield) |
| 30 | A-4 | C-2 | 55% (GC yield) |
| 31 | A-5 | C-2 | 62% (GC yield) |
| 32 | A-6 | C-2 | 55% (GC yield) |
| 33 | A-9 | C-4 | 74% (GC yield) |
| 34 | A-10 | C-5 | 86% (GC yield) |
| 35 | A-11 | C-4 | 52% (GC yield) |

### (Substrates in Examples 8 to 35 (Substrates Given in Tables 2 and 3))

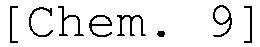

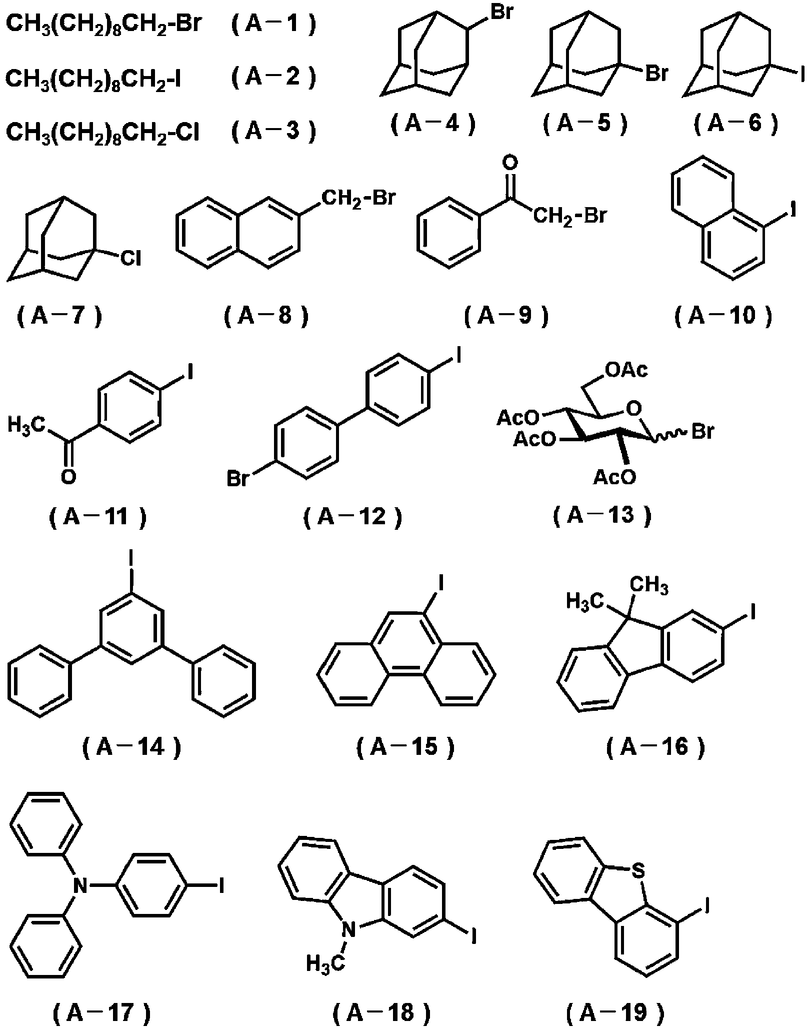

### (Reaction Products in Examples 8 to 35 (Reaction Products Given in Tables 2 and 3))

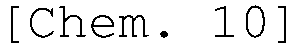

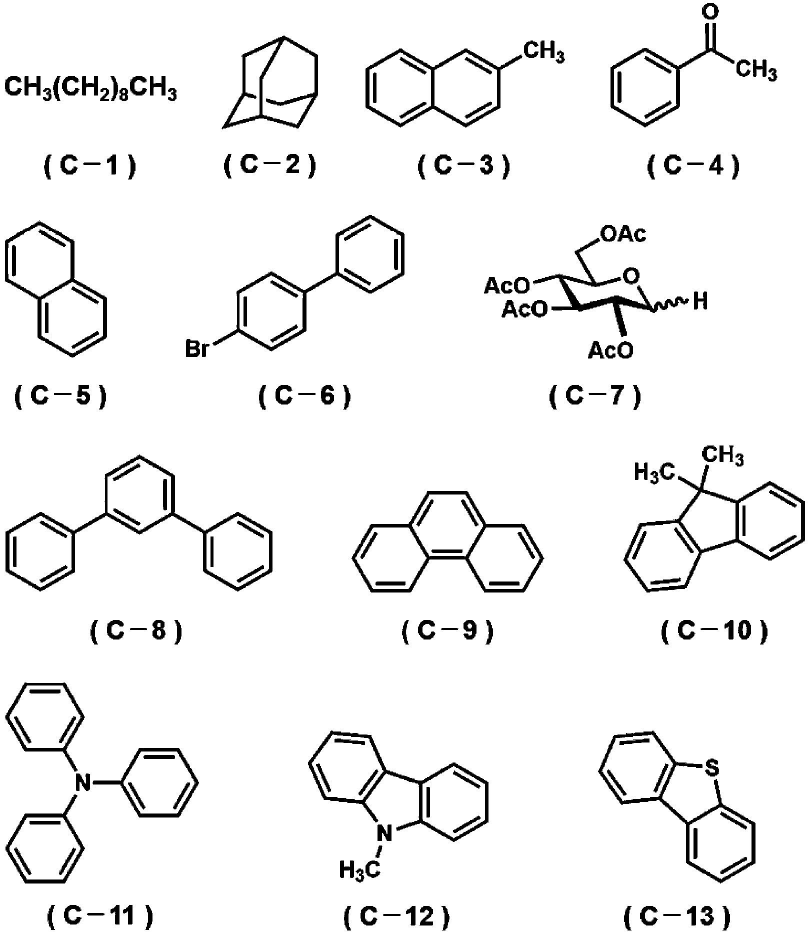

### {Study of Thiol Addition}

### <Example 36>

To a 5-mL stainless steel ball mill jar containing a stainless steel ball having a diameter of 10 mm, 34.1 mg (0.2 mmol) of 1-chloroadamantane (A-7) as a substrate, 119.3 mg (0.48 mmol (2.4 equiv based on the substrate)) of tris(trimethylsilyl)silane as a reducing agent, 300 mg of an ultra-high-molecular-weight polyethylene ("434272-100G" manufactured by Aldrich) (B-1) as an organic polymer material, and 48.6 mg (0.24 mmol) (1.2 equiv based on the substrate)) of 1-dodecanethiol as a thiol compound were added under air. Thereafter, the lid of the ball mill jar was closed. The ball mill jar was attached to a ball mill. The reaction was performed for 60 minutes by shaking and stirring at a vibration frequency of 30 Hz while the ball mill jar was heated from the outside (internal temperature: 60°C) with a heat gun set at 100°C. After the reaction was completed, the mixture was dissolved in ethyl acetate and filtered. The ethyl acetate was then removed using an evaporator. The crude product was purified by silica gel column chromatography to provide adamantane (C-2) as the reaction product. The GC yield of the reaction product was 3%.

### {Study of Reducing Agent}

### <Example 37>

To a 5-mL stainless steel ball mill jar containing a stainless steel ball having a diameter of 10 mm, 50.8 mg (0.2 mmol) of 1-iodonaphthalene (A-10) as a substrate, tri-n-butyltin hydride, as a reducing agent, in an amount of 5 equiv based on the substrate, and 200 mg of polyvinyl acetate (Mn = 3.5 kg/mol, Mw = 8.2 kg/mol (measured by SEC) (B-2)) as an organic polymer material were added under air. The lid of the ball mill jar was closed. Thereafter, the ball mill jar was attached to the ball mill. The reaction was performed by shaking and stirring at a vibration frequency of 30 Hz for 30 minutes while the ball mill jar was heated from the outside with the heat gun set at 250°C (internal temperature: 120°C). After the reaction was completed, the mixture was dissolved in ethyl acetate and filtered. The ethyl acetate was then removed using an evaporator. The crude product was purified by silica gel column chromatography to provide naphthalene (C-5) as the reaction product. The GC yield of the reaction product was 23%.

### [Radical Cyclization Reaction of Organohalogen Compound]

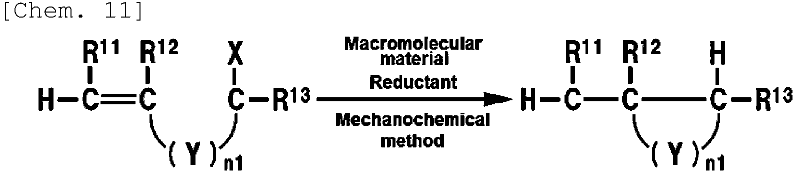

### <Example 38>

To a 5-mL stainless steel ball mill jar containing a stainless steel ball having a diameter of 10 mm, 52.0 mg (0.20 mmol) of 1-bromo-2-(allyloxy)benzene (G-1) as a substrate, 119.3 mg (0.48 mmol (2.4 equiv based on the substrate)) of tris(trimethylsilyl)silane as a reducing agent, and 300 mg of an ultra-high-molecular-weight polyethylene ("434272-100G" manufactured by Aldrich) (B-1) as an organic polymer material were added under air. Thereafter, the lid of the ball mill jar was closed. The ball mill jar was attached to a ball mill. The reaction was performed for 60 minutes by shaking and stirring at a vibration frequency of 30 Hz while the ball mill jar was heated from the outside (internal temperature: 80°C) with a heat gun set at 160°C. After the reaction was completed, the mixture was dissolved in ethyl acetate and filtered. The ethyl acetate was then removed using an evaporator. The crude product was purified by silica gel column chromatography to provide 3-ethyl-2,3-dihydrobenzofuran (H-1) as the reaction product. The isolated yield of the reaction product was 33%.

### <Examples 39 and 40>

Reaction products (H-2) and (H-3) given in Table 4 were obtained in the same manner as in Example 38, except that compounds (G-2) and (G-3) given in Table 4 were used as the substrates. The isolated yield, NMR yield, or GC yield of each reaction product is also given in Table 4.

**[Table 4]**

| Example | Substrate | Reaction product | Yield |
|---|---|---|---|
| 38 | G-1 | H-1 | 33% (GC yield) |
| 39 | G-2 | H-2 | 53% (Isolated yield) |
| | | | 70% (NMR yield) |
| 40 | G-3 | H-3 | 44% (GC yield) |
| | | | d.r. > 95:5 |

### (Substrates in Examples 38 to 40 (Substrates Given in Table 4))

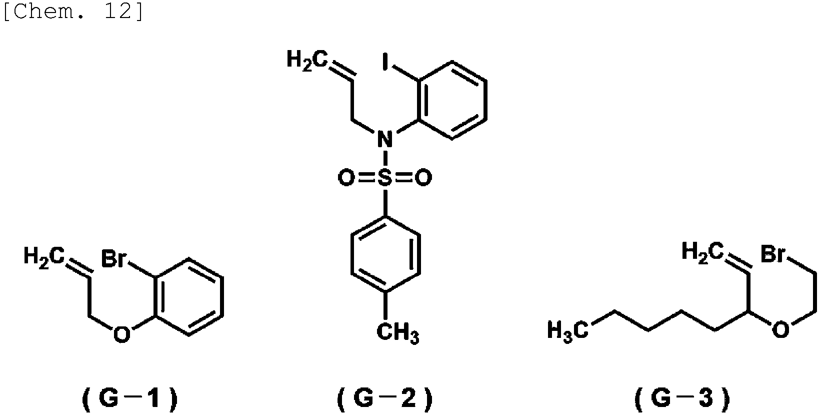

### (Reaction Products in Examples 38 to 40 (Reaction Products Given in Table 4))

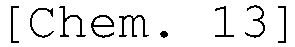

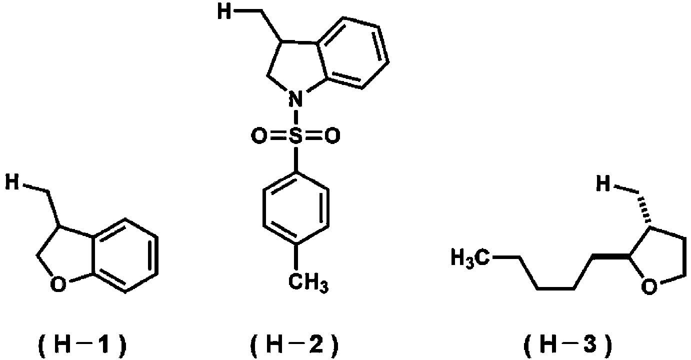

### [Radical Chain Type C-H Fluorination Reaction]

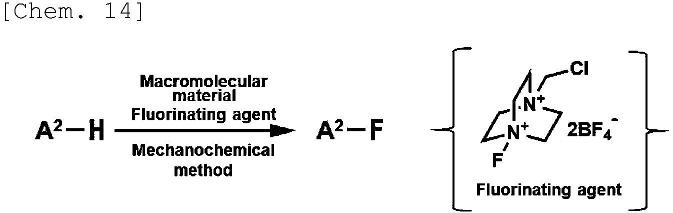

### <Example 41>

To a 5-mL stainless steel ball mill jar containing a stainless steel ball having a diameter of 10 mm, 27.2 mg (0.2 mmol) of adamantane as a substrate, 155.9 mg (0.44 mmol (2.2 equiv based on the substrate)) of 1-chloromethyl-4-fluoro-1,4-diazoniabicyclo[2.2.2]octane bis(tetrafluoroborate) as a fluorinating agent and a reducing agent, 300 mg of an ultra-high-molecular-weight polyethylene ("434272-100G" manufactured by Aldrich) as an organic polymer material, and 0.24 ml (0.5 µl/mg based on the substrate) of acetonitrile as liquid assisted grinding (LAG) were added under air. Thereafter, the lid of the ball mill jar was closed. The ball mill jar was attached to a ball mill. The reaction was performed for 60 minutes by shaking and stirring at a vibration frequency of 30 Hz while the ball mill jar was heated from the outside (internal temperature: 60°C) with a heat gun set at 100°C. After the reaction was completed, the mixture was dissolved in ethyl acetate and filtered. The ethyl acetate was then removed using an evaporator. The crude product was purified by silica gel column chromatography to provide 1-fluoroadamantane as the reaction product. The NMR yield of the reaction product was 17%.

### <Comparative Example 3>

The reaction was performed as in Example 41, except that the ultra-high-molecular-weight polyethylene was not added to the ball mill jar (the reaction was performed without any polymer material). The NMR yield of the reaction product (1-fluoroadamantane) was 2%.

### [Scale-up of Radical Reduction Reaction of Organohalogen Compound]

### <Example 42>

To a 10-mL stainless steel ball mill jar containing a stainless steel ball having a diameter of 15 mm, 538 mg (2.5 mmol) of 1-bromoadamantane (A-5) as a substrate, 1.49 g (6.0 mmol (2.4 equiv based on the substrate)) of tris(trimethylsilyl)silane as a reducing agent, and 3.75 g of an ultra-high-molecular-weight polyethylene ("434272-100G" manufactured by Aldrich) (B-1) as an organic polymer material were added under air. Thereafter, the lid of the ball mill jar was closed. The ball mill jar was attached to a ball mill. The reaction was performed for 60 minutes by shaking and stirring at a vibration frequency of 25 Hz while the ball mill jar was heated from the outside (internal temperature: 80°C) with a heat gun set at 160°C. After the reaction was completed, the mixture was dissolved in ethyl acetate and filtered. The ethyl acetate was then removed using an evaporator. The crude product was purified by silica gel column chromatography to provide adamantane (C-2) as the reaction product. The isolated yield of the reaction product was 85%.

### [Reduction Treatment of Brominated Flame Retardant]

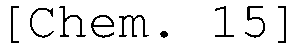

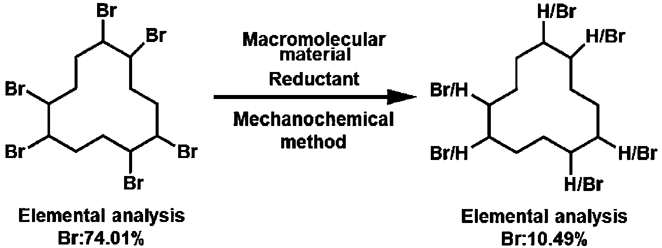

### <Example 43>

To a 5-mL stainless steel ball mill jar containing a stainless steel ball having a diameter of 10 mm, 32.1 mg (0.05 mmol) of 1,2,5,6,9,10-hexabromocyclodecane as a substrate, 124.3 mg (0.5 mmol, 10 equiv based on the substrate) of tris(trimethylsilyl)silane as a reducing agent, and 300 mg of an ultra-high-molecular-weight polyethylene ("434272-100G" manufactured by Aldrich) as an organic polymer material were added under air. Thereafter, the lid of the ball mill jar was closed. The ball mill jar was attached to a ball mill. The reaction was performed for 60 minutes by shaking and stirring at a vibration frequency of 30 Hz while the ball mill jar was heated from the outside (internal temperature: 60°C) with a heat gun set at 100°C. After the reaction was completed, the mixture was dissolved in ethyl acetate and filtered. The ethyl acetate was then removed using an evaporator. The crude product was purified by silica gel column chromatography. The elemental analysis of the reaction product revealed that the bromine content, which had initially been 74.01% (C₁₂H₁₈Br₆), was 10.49% (C₁₂H_{23.75}Br_{0.25}).

### [Reduction Treatment Using Waste Plastic]

### <Example 44>

To a 5-mL stainless steel ball mill jar containing a stainless steel ball having a diameter of 10 mm, 50.8 mg (0.2 mmol) of 1-iodonaphthalene as a substrate, 119.3 mg (0.48 mmol, 2.4 equiv based on the substrate) of tris(trimethylsilyl)silane as a reducing agent, and 300 mg of a waste plastic shopping bag made of polyethylene as an organic polymer material were added under air. The lid of the ball mill jar was closed. Thereafter, the ball mill jar was attached to the ball mill. The reaction was performed by shaking and stirring at a vibration frequency of 30 Hz for 60 minutes while the ball mill jar was heated from the outside with the heat gun set at 100°C (internal temperature: 60°C). After the reaction was completed, the mixture was dissolved in ethyl acetate and filtered. The ethyl acetate was then removed using an evaporator. The crude product was purified by silica gel column chromatography to provide naphthalene as the reaction product. The GC yield of the reaction product was 69%.

## Claims

1. A polymer mechanoradical initiator obtained by mechanochemical treatment of an organic polymer material, wherein the polymer mechanoradical initiator is used in a reaction in which the polymer mechanoradical initiator is not incorporated into a reaction product.

2. The radical initiator according to claim 1, wherein the organic polymer material contains a polyolefin-based material, a polyaromatic vinyl-based material, a polyvinyl ether-based material, a polyvinyl ester-based material, a (meth)acrylic resin-based material, a cellulose-based material, a polyether-based material, a polyester-based material, a polycarbonate-based material, a polyketone-based material, a polyurethane-based material, a polyamide-based material, a polyimide-based material, a polysiloxane-based material, a polyacetal-based material, a modified polyphenylene ether-based material, an ABS resin-based material, and a halogenated olefin-based material.

3. A reaction method for radical reduction and/or radical cyclization of a substrate using a polymer mechanoradical initiator, the method comprising:
a step of performing mechanochemical treatment of at least an organic polymer material and a substrate in a reaction vessel,
wherein the substrate contains an organohalogen compound.

4. A reaction method for C-H fluorination of a substrate using a polymer mechanoradical initiator, the method comprising:
a step of performing mechanochemical treatment of at least an organic polymer material, a substrate, and a fluorinating agent in a reaction vessel,
wherein the substrate contains a compound having a C-H bond based on secondary carbon or tertiary carbon.

5. The reaction method according to claim 3 or 4, wherein the organic polymer material contains a polyolefin-based material, a polyaromatic vinyl-based material, a polyvinyl ether-based material, a polyvinyl ester-based material, a (meth)acrylic resin-based material, a cellulose-based material, a polyether-based material, a polyester-based material, a polycarbonate-based material, a polyketone-based material, a polyurethane-based material, a polyamide-based material, a polyimide-based material, a polysiloxane-based material, a polyacetal-based material, a modified polyphenylene ether-based material, an ABS resin-based material, and a halogenated olefin-based material.

6. The reaction method according to claim 3, wherein the organic polymer material and/or the substrate includes waste.

7. The reaction method according to claim 3 or 6, wherein the reaction method is a radical reduction reaction method, and a reducing agent containing a silane-based reducing agent and/or a tin-based reducing agent is used.
